# EUROPEAN PATENT APPLICATION

(11) **EP 2 491 859 A1**
(43) Date of publication of application: **29.08.2012**
(21) Application number: 11155640.3
(22) Date of filing: 23.02.2011
(51) Int. Cl.: A61B 5/145

(54) **Method and system for determining blood glucose characteristics from a discontinuous mode of measurement and computer program product**

(71) Applicant: F. Hoffmann-La Roche AG, 4070 Basel (CH); Roche Diagnostics GmbH, 68305 Mannheim (DE)
(72) Inventor: Atkinson, Craig Michael, 2086 Australia (AU)
(74) Representative: Bittner, Thomas L.

(57) **Abstract**

The invention relates to a method for determining blood glucose characteristics. The method comprising steps of receiving blood glucose input data representing blood glucose values measured in a discontinuous mode by a blood glucose measurement device (1), receiving time information data representing measurement time information for the blood glucose values, determining time period information representing a time period between a early and a later blood glucose value measured after the early blood glucose value, determining a value difference representing the difference between the early and the later blood glucose value, determining time dependent blood glucose characteristics by deriving time dependent blood glucose change information based on an assignment of the value difference to the time period information, and providing blood glucose characteristics output data representing the time dependent blood glucose characteristics. Furthermore, the invention relates to a system for determining blood glucose characteristics and a computer program product.

## Description

The invention relates to a method and a system for determining blood glucose characteristics from a discontinuous mode of measurement and a computer program product.

### Background

Such methods and systems are used in order to determine characteristics of patients' measured blood glucose values. The objective is to give the patient as well as the attending medical staff information which enable the patient to deal with his blood glucose values in an improved and appropriate manner.

For people suffering from diabetes, in particular Diabetes Mellitus, it is especially important for them to keep their blood glucose values constantly at a particular level. A precondition for this is knowledge of their blood glucose value which is therefore measured using a blood glucose measuring device set up for this purpose. Blood glucose measuring devices are known in various embodiments.

If it is determined, on the basis of the measured values, that the blood glucose value has exceeded the recommended level, medicine is administered, for example by means of insulin injection or the oral administration of Metformin, an oral antidiabeticum. If the blood glucose values fall below the ideal or recommended level, sugar must be orally ingested, for example through food or drink. If the ideal level is exceeded for an extended period of time, there is the danger of serious health complications such as blindness, kidney damage, limbs having to be amputated or neuropathy. If the exceeding of the prescribed blood glucose level is for a short time only but considerable, this can lead to nausea, dizziness, sweating or even conditions of confusion. Thus, it is particularly important for a diabetic to know his blood glucose values at all times so that he is able to implement the appropriate measures to avoid the blood sugar values deviating from the ideal levels.

A blood glucose measuring device with which the blood glucose values of diabetics can be measured is known, for example from the document DE 10 2004 057 503 A1 and is sold by the applicant under the registered trade mark, Accu-Chek.

It is known, blood glucose measurements should be made according to a continuous measurement regime. Such measurements are also known as CGM measurements (Continuous Monitoring blood Glucose Measurement). In this process, the blood glucose values are measured continuously in a continuous time period such that, for example, the progress of the blood glucose value can be collected over an entire day. The analysis of the measured blood glucose values can provide for the determination of several day trends. In this way it is possible to determine blood glucose fluctuations dependent on the time of day. A disadvantage of the continuous blood glucose measurements is firstly, due to the continuous measurements, it can lead to high costs and secondly that it leads to discomfort for the diabetic whereby the latter is caused by the permanent wearing of a subcutaneous sensor. This can lead to infections at the point of entry, intolerance of the plaster or skin irritation which prevents the device being worn permanently or for a long period of time, for example several months. A continuous measurement of the blood glucose values is described, for example, in the document, Gross et al., "Performance Evaluation of the MiniMed Continuous Glucose Monitoring System During Patient Home Use", Diabetes Technology & Therapeutics, 2 (2000)49.

In connection with the continuous monitoring of blood glucose values, the proposal is made in the document US 2008 / 0287755 A1, to perform a so-called trend analysis. This can enable the rate of change to be determined on the basis of two or more blood glucose value measurements. The period of continuously monitoring can be between 5 and 30 minutes. Measurement periods of less than 10 minutes or more than 30 minutes can be provided. Within the measurement period, the CGM measurement is performed once per second or once per minute whereby constant or variable cycle lengths can be provided. The known method proposes that the trend thus determined can be presented on a display as a directional arrow.

Furthermore, discontinuous or structured blood glucose measurements are known which are especially also known as SMBG measurements (Self Monitoring Blood Glucose Measurement) and are characterised by the fact that blood glucose values are determined at particular time intervals by means of individual measurements and / or series of measurements. In this way, it is possible, with the help of such blood glucose measurements, which are also known as structured blood glucose measurements, to measure blood glucose values in close proximity to particular events, for example in relation to meals. Disadvantages of structured blood glucose measurement can arise if events relevant for the blood glucose value of the diabetic occur between the measurement times so that they are undetectable. In the document US 2009 / 0054753 A1, a procedure is described whereby an individual blood glucose measurement in the scope of a discontinuous measurement is used to determine the time for a subsequent further individual measurement according to certain parameters. The parameters take into account patient and environmental conditions.

In connection with the discontinuous determination of blood glucose values, it was further proposed to determine the difference between pre and postprandial blood glucose values. This involves blood glucose values being evaluated before and after a meal by determining the dif ference between the pre and postprandial blood glucose values.

It is also known, in connection with other events, to determine their influence on blood glucose values. It can be provided that details of the event, for example the type of meal or type of physical activity, can be incorporated in the evaluation of the blood glucose levels.

### Summary of the invention

It is the object of the invention to provide improved technologies for determining blood glucose characteristics derived from a discontinuous mode of measurement.

According to the invention a method and a system for determining blood glucose characteristics from a discontinuous mode of measurement according to claim 1 and 10 respectively, are provided. In addition, a computer program product according to claim 11 is provided. Advantageous developments of the invention are disclosed in dependent claims.

According to one aspect of the invention, a method for determining blood glucose characteristics from a discontinuous mode of measurement is provided, in a data processing system the method comprising steps of:
- receiving blood glucose input data representing blood glucose values measured in a discontinuous mode of measurement by means of a blood glucose measurement device,
- receiving time information data representing measurement time information for the blood glucose values,
- determining time period information representing a time period between at least one early blood glucose value and at least one later blood glucose value measured timely after the measurement of the at least one early blood glucose value,
- determining a value difference representing the difference between the at least one early blood glucose value and the at least one later blood glucose value,
- determining time dependent blood glucose characteristics by deriving time dependent blood glucose change information based on an assignment of the value difference to the time period information, and
- providing blood glucose characteristics output data representing the time dependent blood glucose characteristics.

According to another aspect of the invention, a system for determining blood glucose characteristics is provided, the system comprising a data processing system and a computer program code configured for the above method for determining blood glucose characteristics. In a preferred embodiment, the blood glucose measurement device itself is configured to implement the method for determining blood glucose characteristics.

According to still another aspect of the invention, a computer program product is provided, preferably stored on a storage medium and configured to perform the method for determining blood glucose characteristics during operation on a data processing device.

With the help of the invention, it is possible, in the scope of discontinuous blood glucose measurements to provide the patient and/or medical staff information as to the time related changes of the measured blood glucose values. The time related changes can show an increase, decrease or constant level of blood glucose values. The information about the time related trend of blood glucose values can preferably be determined in the form of a positive or negative slope which would equate to an increase or decrease. If not time related change in the blood glucose levels is determined, the slope is flat. The blood glucose characteristics output data can be provided in the form of text and acoustic data. Signal data can also be provided which would lead to a video and/or audio output.

The provision of time related increase, or trend information in the scope of discontinuous blood glucose value measurements, gives the patient or the medical staff the opportunity to react to time related changes appropriately. The invention thereby provides in particular a trend analysis, which in the prior art is only known for continuous blood glucose measurement systems. Devices implementing a continuous blood glucose measurement method, however, are not appreciated by many patients, because of high costs. In addition, the necessity of continuous subcutan measurements sometimes not accepted by the patient. By the invention a method and a system for determining blood glucose characteristics exclusively from a discontinuous mode of measurement are provided. In one embodiment, it is provided to analyse the time dependent trend, prior to an event, for example a physical activity or a meal, in order to determine, on the basis of that information, for example, an insulin dose.

It is an additional advantage of providing a trend information to the patient or the medical stuff that - in contrast to only calculating a value difference between two blood glucose measurements at different times - the patient will learn whether there is a stable or unstable blood glucose value course. Especially, a situation characterized by a strongly decreasing blood glucose trend can be avoided.

A further development of the invention is that at least one early blood glucose value and at least one later blood glucose value are processed for which the time period is shorter than 2.0 hours, preferred shorter than 1.0 hour, and further preferred shorter than 0.5 hours. The setting of the time period means that within the set time periods no blood glucose levels are measured and therefore not evaluated. The point is, therefore, to determine the time dependent trend with the help of one or more measurement values at the beginning of the period and one or more measurement values at the end of the period. The proposed procedure differs from the continuous monitoring blood glucose measurement in which measurements over longer time periods are also taken, whereby in this case continuous measurements are also taken within the time period so that the pattern over longer periods is overlayed by pattern over shorter periods. In contrast, the procedure proposed here provides a time dependent trend analysis for blood glucose values at the beginning and at the end of a time period whereby the time interval between the beginning and the end of the period is preferred to be at least 0.3 hours, further preferred to be at least 0.5 hours and still further preferred at least 1 hour. With the discontinuous mode of measurement the risk may arise that an increase between two measurement values is evaluated, for which the measurement events are separated too far in time. Therefore, the trend information derived from such analysis will not indicate the real trend of the blood glucose characteristic. If, for example, the trend information is derived from a blood glucose value which decreases and which will increase following a wrong trend information is provided if measurement values are only analyzed for first and last measurements during the time period. The system for determining blood glucose characteristics, therefore, will provide a non-correct trend information to the patient, namely information about a decreasing blood glucose value. However, in reality there is an increase of the blood glucose value. Therefore, in such situation there is a risk of having the patient or the medical stuff initiated a wrong care taking step which will lead to some health risk for the patient. Such situation can be avoided by the above embodiment of the invention which will ensure that correct trend information derived from to measurements is provided.

In a preferred embodiment, the method further comprises steps of providing an upper time limit parameter, and cancelling further processing when in the step of determining the time period information a time period is determined which exceeds the upper time limit parameter. In this embodiment, the further processing of blood glucose values is cancelled if during the determining of the time period information, it is noticed that the time period determined, between the at least one early blood glucose value and the at least one later blood glucose value, exceeds an upper time limit parameter. If it is determined that the time interval between the blood glucose values to be analysed is too long, then no determination of the time dependent trend of the blood glucose value is performed. In this way, one can prevent the time dependent trend analysis, in the scope of the discontinuous blood glucose value measurement being performed for time periods within which, according to experience, a fluctuation of the blood glucose level takes place so that performing a time dependent determination of the trend with the help of blood glucose values at the beginning and end of the period would not be meaningful.

In another preferred embodiment, the method further comprises a step of providing cancelling information for output. In this embodiment, the user is informed of an automatic cancelling of the process of blood glucose value processing, in particular in cases where the time interval between the at least one early blood glucose value and the at least one later blood glucose value measurements exceeds the prescribed time limit whereby the user is given the opportunity to reinitiate the process and possibly with other parameters. For example, the user can reduce the upper time limit parameter.

In an advanced embodiment of the invention, the method further comprises steps of receiving event information representing an event potentially having an impact on the measured blood glucose values, comparing the event information to event related upper time limit information, and adjusting the upper time limit parameter. The user is, for example, given the opportunity to input information relating to a particular event, so that the upper time limit parameter is set accordingly. This can be realised, for example, in that a dedicated storage unit on which is stored data relating to the upper time limit parameter for different types of event, such as sporting activity or types of meal, from which, dependent on the user input received, the upper time limit parameter can be set in relation to a particular event. For this, the data processing system accesses a respective storage unit.

Preferably, the step of determining the value difference comprises a step of determining the difference between at least a pre-event blood glucose value and at least a post-event blood glucose value. In this embodiment, the time dependent trend for the blood glucose value is determined for a time period within which an event lies, which can potentially influence the blood glucose value. For example, a pre-prandial and post-prandial blood glucose value can be consulted for the analysis, that is to say measurement values before and after a meal. This enables, generally, a time dependent trend analysis in connection with a particular event. Alternatively, or additionally it can be provided that the time dependent trend analysis is determined for blood glucose values which all lie before or after such an event whereby a trend before or after an event can be determined.

A further development of the invention is that the step of determining the value difference is performed by processing an average value for at least one of the at least one early blood glucose value and the at least one later blood glucose value. In this embodiment, the at least one early blood glucose value and / or the at least one later blood glucose value determined from several blood glucose values which are allocated to the at least one early blood glucose value or the at least one later blood glucose value measurement and collected respectively in one time period which is comparatively shorter than the time interval between the collection of the at least one early blood glucose value and the at least one later blood glucose measurement values. For example, several blood glucose values are collected in an interval of 30 seconds or 1 minute, whereby the time interval between the at least one early blood glucose value and the at least one later blood glucose (mean) values is at least 0.5 hours, preferred at least 1 hour and further preferred at least 2 hours.

In a further preferred embodiment, the method further comprises a step of providing patient warning information if the time dependent blood glucose change information indicates a time dependent blood glucose change outside a given change limit. The change limit has been provided before in that, for example, a respective user input is collected. Alternatively, or additionally, it can be provided that data pertaining to change limits is stored in a designated storage unit which can be tailored according to the specific patient. The patient warning information can be communicated with the help of video and / or audio signals. For example, the user can be shown that a planned physical activity should be abandoned if the time dependent trend information determined prior to the planned event indicates this. The user can also be shown that a dose of insulin would be appropriate if this is indicated by the time dependent trend determined. The level at which a blood glucose change will initiate patient warning information may depend on an event happened before. For example, device input information about a physical activity and / or food intake of the patient may be received. Depending on the characteristic of the event, blood glucose change limits may be set for initiating the patient warning information.

In still a further preferred embodiment, the method further comprises steps of comparing the time dependent blood glucose change information to insulin bolus information, and providing insulin bolus adjustment information for output, if a need for adjustment is concluded from comparing the time dependent blood glucose change information to insulin bolus information. In this connection, it can be provided that the insulin bolus adjustment information shows the user he should deviate from a previously given bolus information as a particular time dependent trend for blood glucose has been determined.

In another preferred embodiment, the method further comprises a step of providing further event information. For example, such event information will propose a future blood glucose measurement event to the patient or the medical stuff in dependence on the blood glucose analysis performed before. Such event information may comprise a date and / or time for the future measurement event. As an alternative, a time period may be provided, such time period information indicating a time period which has to be added to the last measurement event before the future measurement event has to be done. In addition or as an alternative, the event information may provide a request to the patient or the medical stuff for taking specific action, for example food intake.

### Description of preferred embodiments of the invention

In the following, the invention will be described in further detail, by way of example, with ref erence to different embodiments. The figures show:
- Fig. 1: a schematic representation of a system for affording a method for determining blood glucose characteristics,
- Fig. 2: a schematic presentation of blood glucose value measurements,
- Fig. 3: a schematic representation of a display of a blood glucose measurement device present-ing a menu for setting up a trend analysis, and
- Fig. 4: several screens of a blood glucose measurement device presenting different results of a trend analysis.

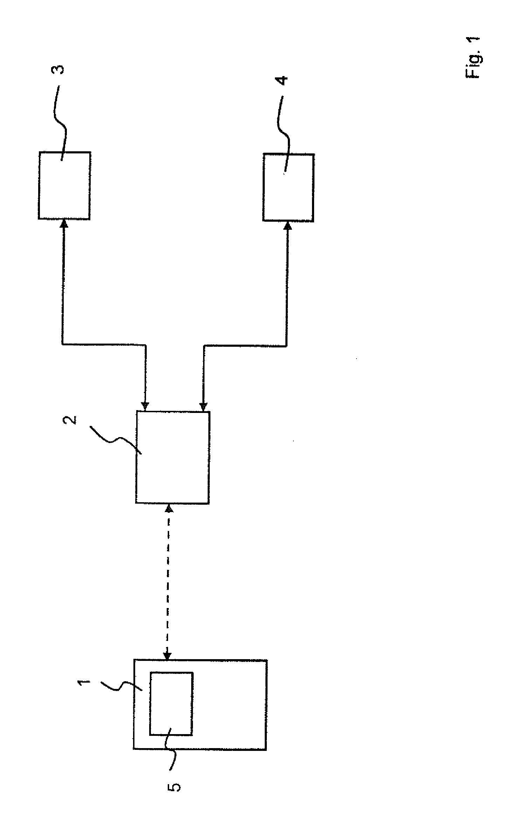

Fig. 1 shows a schematic representation of a system for determining blood glucose characteristics from a discontinuous mode of measurement, the system comprising a blood glucose measurement device 1, a data processing system 2 connectable to the blood glucose measurement device 1, an output device 3, and a data base 4. The data processing system 2 may be provided as a computer device, e.g. a so-called all-purpose computer.

The elements shown in Fig. 1 may be provided in a network system wherein network elements are connected by wireless and / wired data transmission connections.

In a preferred embodiment, the data processing system 2, the output device 3, and the data base 4 may be implemented at least in part in the blood glucose measurement device 1 itself. For example, the data processing system 2 and the output device 3 may be implemented in the blood glucose measurement device 1 itself, but the data base 4 may be provided in a central server device which is connected to the blood glucose measurement device 1 by a wireless data connection, for example via the Internet. In an embodiment, the output device 3 may be implemented by a display 5 of the blood glucose measurement device 1.

The procedure described in the following, can be embodied in the above explanations on the different embodiment variations.

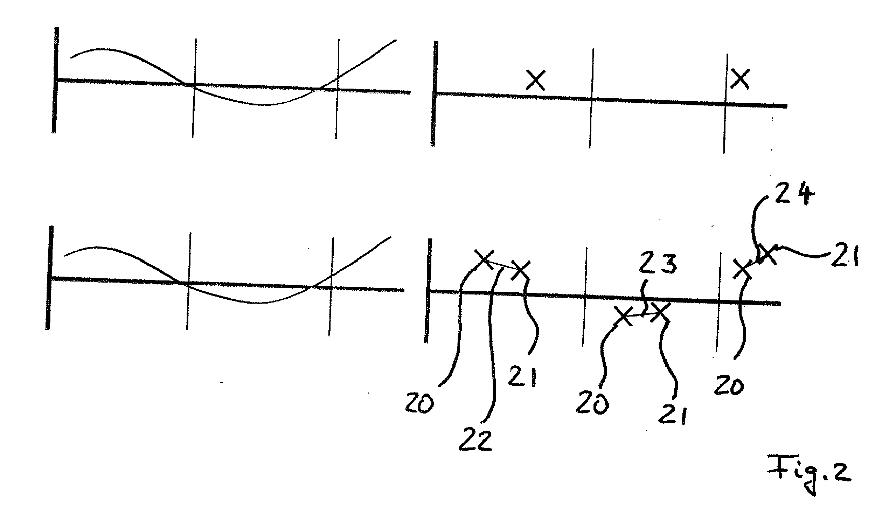

Fig. 2 shows the schematic representation of measured blood glucose values (y-axis) dependent on time (x-axis).

On the left hand side of Fig. 2, time dependent continuous trend of blood glucose measurement values known in the art are shown. For example, these trends show the increase and decrease of the continuously collected blood glucose values dependent on the time.

On the right hand side of Fig. 2, the upper curve shows the standard procedure for the discontinuous determination of the blood glucose values. Throughout the day, in larger time intervals, for example of several hours, blood glucose values are determined which is diagrammatically shown in Fig. 2 with the help of crosses.

On the right hand side of Fig. 2, the lower curve shows that an at least one early blood glucose value and an at least one later blood glucose value 20, 21 are determined. For a time period between the at least one at least one early blood glucose value blood glucose value and the at least one later blood glucose value 20, 21 a time dependent trend or slope is determined in which the difference between the at least one early blood glucose value and the at least one later blood glucose value 20, 21 is shown in relation to the time interval between the two blood glucose value 20, 21.

In this way, a rate of change (slope or trend), that is to say a slope in relation to time which can be positive or negative for the time period between the at least one early blood glucose value and the at least one later blood glucose value. It can be provided that in the time interval between the at least one early blood glucose value and the at least one later blood glucose value 20, 21 lies an event, for example a meal or physical activity. It can, however, be provided that the measurement of the at least one early blood glucose value and the at least one later blood glucose value 20, 21 can occur before or after such an event.

In Fig. 2, the trend or slope for a first time period 22 is negative and for a second and third time periods 23, 24 positive which corresponds to a decrease in blood glucose values in the first time period 22 and an increase in the second and third periods 23, 24 whereby the increase in the third period 24 is greater than that in the second time period 23.

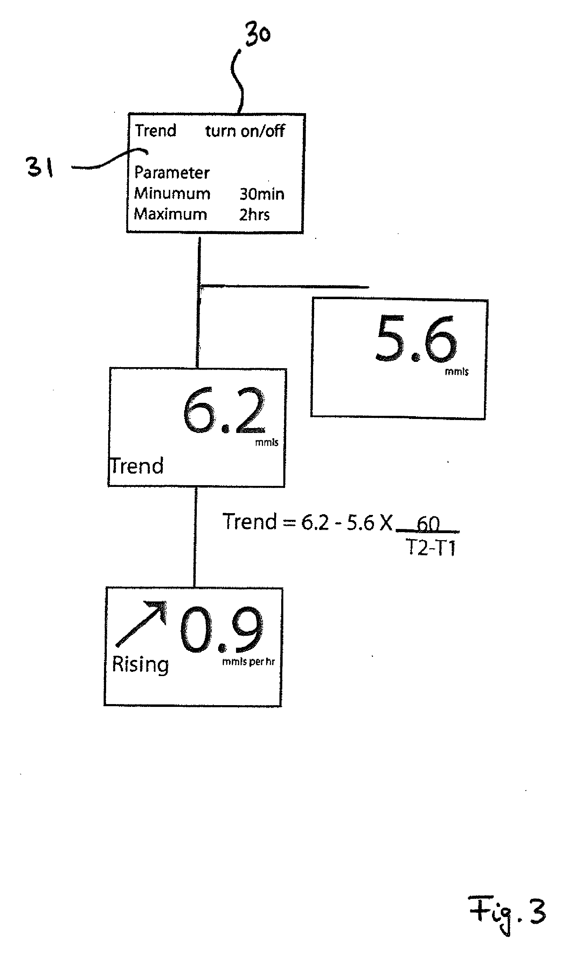

Fig. 3 shows a schematic representation of a screen 30 of the blood glucose measurement device 1 with a user menu 31 relating to the trend analysis for the blood glucose values in the scope of the discontinuous blood glucose value determination. The representation refers to the embodiment of the system in which the data processing system 2 is integrated into the blood glucose measurement device 1. The user is given the opportunity of activating or deactivating the time dependent trend analysis. Furthermore, Fig. 3 shows the parameters set for the minimum (0.5 hours) and maximum (2 hours) time periods for which the time dependent trend / slope is determined. With the help of respective user inputs, the user of the blood glucose measurement device 1 can set these limits.

In Fig. 3, the screen 30 of the blood glucose measurement device 1 is shown after a first measurement (5.6 mmls) and a second measurement (6.2 mmls). In conclusion, there is a positive slope of 0.9 mmls per hour (rate of change).

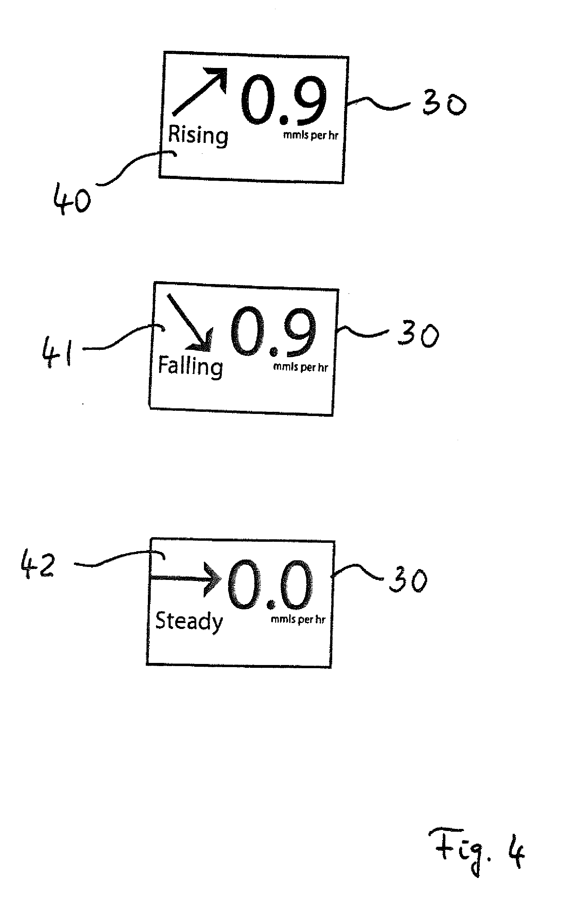

Fig. 4 shows a schematic representation of the display or screen 30 of the blood glucose measurement device 1 of Fig. 1, whereby various results of the time dependent trend analysis are shown, namely an increase 40 (positive slope - rising), a decrease 41 (negative slope - falling) as well as an unchanged trend 42 (steady).

In the following, further aspects of the invention are described by referring to different situations to which the invention may apply.

A person A with diabetes would like to evaluate their basal rate prior to their breakfast meal. Measurements are taken at 7.50 a.m. providing an earlier blood glucose value of 6.7 mmls and again at 8.43 a.m. providing a later blood glucose value of 9.3 mmls. The method described above will provide the information that the blood glucose value is rising at a high rate. Person A should consult with their clinical team to maybe adjust basal insulin to correct the morning rise in blood glucose.

In another situation, a person B would like to optimise their sporting performance by maintaining a blood glucose level of about 8 mmls throughout a physical activity. Person B tests in a discontinuous mode of measurement twice before the activity, twice during the activity and twice after the activity. The method described above will show person B before the activity their blood glucose is falling at 1.8 mmls per hour (negative slope), during the activity their blood glucose is rising at 1.7 mmls per hour (positive slope) and after the activity the blood glucose is rising at 3.2 mmls per hour (positive slope again). Person B consults with the clinical team and discovers the activity is causing stress with person B and does not display the typical expectation that glucose level will fall with activity. The carbohydrates taken before the exercise are increasing the blood glucose level too rapidly for peak performance.

In still another situation, a person C is afraid of nocturnal hypoglycaemia and prefers to have night time blood glucose levels at about 8 mmls to prevent these events, this in turn has raised person C to a higher HbA1C level. With the use of the method described above, person C is happy to go to bed with a blood glucose value of 6 mmls if they have tested prior to calculate that their blood glucose level is stable. In general, the reassurance of a level line gives the patients confidence in keeping their blood glucose at an optimum level to reduce the complications of diabetes.

The features disclosed in this specification, the figures and / or the claims may be material for the realization of the invention in its various embodiments, taken in isolation or in various combinations thereof.

## Claims

1. A method for determining blood glucose characteristics from a discontinuous mode of measurement, in a data processing system (2) the method comprising steps of:
- receiving blood glucose input data representing blood glucose values measured in a discontinuous mode of measurement by means of a blood glucose measurement device (1),
- receiving time information data representing measurement time information for the blood glucose values,
- determining time period information representing a time period between at least one early blood glucose value and at least one later blood glucose value measured timely after the measurement of the at least one early blood glucose value,
- determining a value difference representing the difference between the at least one early blood glucose value and the at least one later blood glucose value,
- determining time dependent blood glucose characteristics by deriving time dependent blood glucose change information based on an assignment of the value difference to the time period information, and
- providing blood glucose characteristics output data representing the time dependent blood glucose characteristics.

2. The method according to claim 1, wherein at least one early blood glucose value and at least one later blood glucose value are processed for which the time period is shorter than 2.0h, preferred shorter than 1.0h, and further preferred shorter than 0.5h.

3. The method according to claim 1 or 2, the method further comprising steps of:
- providing an upper time limit parameter, and
- canceling further processing when in the step of determining the time period information a time period is determined which exceeds the upper time limit parameter.

4. The method according to claim 3, the method further comprising a step of providing canceling information for output.

5. The method according to claims 3 or 4, the method further comprising steps of:
- receiving event information representing an event having potentially in impact on the measured blood glucose values,
- comparing the event information to event related upper time limit information, and
- adjusting the upper time limit parameter.

6. The method according to at least one of the preceding claims, wherein the step of determining the value difference comprises a step of determining the difference between at least a pre-event blood glucose value and at least a post-event blood glucose value.

7. The method according to at least one of the preceding claims, wherein the step of determining the value difference is performed by processing an average value for at least one of the at least one early blood glucose value and the at least one later blood glucose value.

8. The method according to at least one of the preceding claims, the method further comprising a step of providing patient warning information if the time dependent blood glucose change information indicates a time dependent blood glucose change outside a given change limit.

9. The method according to at least one of the preceding claims, the method further comprising steps of:
- comparing the time dependent blood glucose change information to insulin bolus information, and
- providing insulin bolus adjustment information for output, if a need for adjustment is concluded from comparing the time dependent blood glucose change information to insulin bolus information.

10. A system for determining blood glucose characteristics from a discontinuous mode of measurement, comprising a data processing system (2) and computer program code configured for a method according to at least one of the preceding claims.

11. Computer program product, preferably stored on a storage medium and configured to perform the method according to at least one of the claims 1 to 9 during operation on a data processing device (2).
